(19) Europäisches Patentamt
European Patent Office
Office européen des brevets

(11) **EP 1 748 762 B1**

(12) **EUROPÄISCHE PATENTSCHRIFT**

(45) Veröffentlichungstag und Bekanntmachung des Hinweises auf die Patenterteilung:
**18.12.2013 Patentblatt 2013/51**

(51) Int Cl.:
***A61K 9/20*** *(2006.01)*

(21) Anmeldenummer: **05748281.2**

(22) Anmeldetag: **30.05.2005**

(86) Internationale Anmeldenummer:
**PCT/EP2005/005806**

(87) Internationale Veröffentlichungsnummer:
**WO 2005/115343 (08.12.2005 Gazette 2005/49)**

(54) **DOSIERUNGSFORM, ERHÄLTLICH AUS EINER EIN ANORGANISCHES PIGMENT UMFASSENDEN PULVERMISCHUNG**

DOSAGE FORM OBTAINABLE FROM A MIXTURE OF POWDERS CONTAINING AN INORGANIC PIGMENT

FORME DE DOSAGE OBTENSIBLE À PARTIR D'UN MÉLANGE DE POUDRES CONTENANT UN PIGMENT INORGANIQUE

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität: **28.05.2004 EP 04012791**

(43) Veröffentlichungstag der Anmeldung:
**07.02.2007 Patentblatt 2007/06**

(73) Patentinhaber: **AbbVie Deutschland GmbH & Co KG**
**65205 Wiesbaden (DE)**

(72) Erfinder:
• **FASTNACHT, Katja**
**68309 Mannheim (DE)**
• **DEGENHARDT, Matthias**
**67071 Ludwigshafen-Ruchheim (DE)**
• **BERNDL, Gunther**
**67273 Herxheim (DE)**
• **ROSENBERG, Jörg**
**67158 Ellerstadt (DE)**
• **BREITENBACH, Jörg**
**68199 Mannheim (DE)**

(74) Vertreter: **Reitstötter - Kinzebach**
**Patentanwälte**
**Sternwartstrasse 4**
**81679 München (DE)**

(56) Entgegenhaltungen:
**WO-A-01/34121 WO-A-98/20863**
**DE-A- 3 325 130**

**Beschreibung**

Die vorliegende Erfindung betrifft eine feste Dosierungsform, die aus einer eine Kombination aus hochdisperser Kieselsäure und einem anorganischen Pigment umfassenden Pulvermischung erhältlich ist, und ein Verfahren zur Herstellung der Dosierungsform.

Die Herstellung fester Dosierungsformen durch Schmelzextrusion, d. h. ein Verfahren, bei dem man eine Schmelze aus einem polymeren Bindemittel und einem Wirkstoff extrudiert und den extrudierten Strang zu der gewünschten Arzneiform formt, ist bekannt, siehe z. B. EP-A 240 904, EP-A 240 906, EP-A 337 256 und EP-A 358 105. Dieses Verfahren gestattet die Zubereitung schwerlöslicher Wirkstoffe in Form fester Lösungen. In den festen Lösungen liegt der Wirkstoff in molekular gelöster Form vor und kann daher leichter resorbiert werden als der kristalline Wirkstoff.

Bei der Extrusion sind die Bestandteile der Schmelze kurzzeitig einer nicht unbeachtlichen thermischen Belastung ausgesetzt, die zu einer sehr geringfügigen Zersetzung des polymeren Bindemittels und/oder der anderen Bestandteile und einer Verfärbung des Extrudats führen kann. Die Verfärbungen sind zwar unbedenklich, beeinträchtigen aber das optische Erscheinungsbild der erhaltenen Dosierungsformen. Üblicherweise werden die Verfärbungen durch Anbringen eines Filmüberzugs auf der Dosierungsform, z. B. durch Aufsprühen einer Lösung eines filmbildenden Polymers, verdeckt. Durch den Kontakt mit Wasser oder einem anderen Lösungsmittel beim Aufsprühen der Beschichtungslösung kann aber an der Oberfläche der Dosierungsform eine teilweise Rekristallisation des Wirkstoffs induziert werden, mit der Folge, dass sich das Freisetzungsverhalten der Dosierungsform in unkontrollierter Weise verändert.

Bei der Schmelzextrusion wird üblicherweise eine Pulvermischung des Bindemittels, des Wirkstoffs und gegebenenfalls weiterer Komponenten in den Einfülltrichter eines geeigneten Kneters oder Extruders eingefüllt. Für eine problemlose Herstellung und eine genaue volumetrische Dosierung ist ein gutes Fließverhalten der Pulvermischung wünschenswert. Die Pulvermischungen neigen jedoch vielfach aufgrund der Adhäsion der einzelnen Bindemittel-Partikel untereinander zur Brückenbildung und einem ungleichmäßigen Fließverhalten.

Zimmermann, I. et al. berichten in Powder Technology 139 (2004) 40-54 über den Einfluss verschiedener Fließhilfsmittel auf das Fließverhalten von Maisstärke.

Die WO 98/20863 offenbart ein Verfahren zum Beschichten eines Substrats mit den Schritten: Auftragen eines aktiven Überzugsmaterials auf das Substrat unter Erhalt einer aktiven Überzugsschicht, wobei das aktive Überzugsmaterial biologisch aktives Material enthält und die aktive Überzugsschicht vom Substrat entfernbar ist. Das aktive Überzugsmaterial kann in Form eines pulverförmigen Materials eingesetzt werden, das z. B: aus Xylitol, Dilthiazem HCl, $TiO_2$ und kolloidaler Siliciumdioxid zusammengesetzt sein kann.

Die WO 01/34121 offenbart ein Verfahren zur Herstellung einer mehrschichtigem filmförmige Zubereitung aus hydrophilen Polymeren, das unter anderem die Herstellung einer Polymerschmelze umfasst. Die Polymerschmelze enthält Wirkstoffe und gegebenenfalls Hilfs- oder Zusatzstoffe. Als Hilfs- oder Zusatzstoffe sind z.B. Weichmacher, Pigmente, Zerfallsförderer, Netzmittel, resorptions- oder permeationsfördernde Substanzen, Strukturgeber und Texturmodifizierer genannt.

Die DE 33 25 130 A1 offenbart eine feste pharmazeutische Zusammensetzung zur Anwendung für die Haut, gekennzeichnet durch einen Gehalt von 5 bis 35% der Gesamtmasse an essentiellen Fettsäuren auf einem geeigneten festen makromolekularen Träger; Diffusionsregulatoren für die aktiven Prinzipien; Stabilisatoren für als Träger dienendes Kunststoffmaterial und stabilisierende Mittel für die essentiellen Fettsäuren.

Die WO 92/15285 beschreibt Depot-Formulierungen, die eine prozessierte Stärke enthalten. Die Stärke wird in einem Extruder unter scherenden Bedingungen bei Temperaturen von 80 bis 240 °C prozessiert. Dabei können Füllstoffe wie Titandioxid mitverwendet werden.

Die US 5,427,614 offenbart Stärkeformulierungen für den Spritzguss, die Stärke, ein Gleitmittel, ein Flussmittel, ein Texturierungsmittel und Wasser enthalten. Als Texturierungsmittel sind z. B. Kombinationen von Siliciumdioxid und Titandioxid angesprochen. Die Stärkeformulierungen enthalten keinen Wirkstoff; eine Anwendung der Stärkeformulierungen als Dosierungsformen ist nicht vorgesehen.

Der Erfindung liegt die Aufgabe zu Grunde, die oben geschilderten Probleme der Schmelzextrusion zu vermeiden. Insbesondere liegt der Erfindung die Aufgabe zu Grunde, eine durch Schmelzextrusion herstellbare Dosierungsform bereitzustellen, die über eine rasche Wirkstofffreisetzung verfügt, ein optisch ansprechendes Erscheinungsbild ohne das Erfordernis eines Filmüberzugs aufweist und in effizienter Weise herstellbar ist.

Es wurde nun gefunden, dass die Mitverwendung anorganischer Pigmente in der Pulvermischung nicht nur zu einem besseren Fließverhalten der Pulvermischung und damit zu einem besseren Einzugsverhalten im Einfülltrichter führt. Überraschenderweise bewirkt die Mitverwendung der anorganischen Pigmente auch eine raschere Wirkstofffreisetzung aus den erhaltenen Dosierungsformen und verleiht den Dosierungsformen ein optisch ansprechendes Erscheinungsbild auch ohne zusätzlichen Filmüberzug.

Gegenstand der vorliegenden Erfindung ist eine feste Dosierungsform mit einer Matrix mit wenigstens einem homogen in der Matrix dispergierten Wirkstoff, die durch Schmelzen einer Pulvermischung erhältlich ist, wobei die Pulvermischung wenigstens ein thermoplastisches Bindemittel, eine Kombination aus hochdisperser Kieselsäure und wenigstens einem anorganischen Pigment und außerdem wenigstens einen Solubilisator enthält, der unter Sorbitanfettsäureestern, polyalkoxylierten Fettsäureestern oder polyalkoxylierten Ethern von Fettalkoholen ausgewählt ist.

In der erfindungsgemäßen Dosierungsform liegt der Wirkstoff vorzugsweise im Wesentlichen in amorpher Form vor, erkennbar an der Abwesenheit von Beugungsmaxima im Röntgenbeugungsspektrum, die dem kristallinen Wirkstoff zuzuschreiben sind. Besonders bevorzugt liegt der Wirkstoff in der Matrix in Form einer festen Lösung vor.

Die Pulvermischung zur Herstellung bevorzugter Dosierungsformen umfasst:

1,0 bis 30 Gew.-% (vorzugsweise 2,5 bis 20 Gew.-%) Wirkstoff,
0 bis 20 Gew.-% (vorzugsweise 0,1 bis 15 Gew.-%) Solubilisator,
0,5 bis 2,0 Gew.-% (vorzugsweise 0,8 bis 1,5 Gew.-%) hochdisperse Kieselsäure,
0,8 bis 5,0 Gew.-% (vorzugsweise 1,0 bis 4,0 Gew.-%) anorganisches Pigment, und 45 bis 97 Gew.-% thermoplastisches Bindemittel.

Die Erfindung betrifft außerdem ein Verfahren zur Herstellung fester Dosierungsformen, bei dem man

a) eine Pulvermischung bereitstellt, die wenigstens ein thermoplastisches Bindemittel, eine Kombination aus hochdisperser Kieselsäure und wenigstens einem anorganischen Pigment und außerdem wenigstens einen Solubilisator enthält, der unter Sorbitanfettsäureestern, polyalkoxylierten Fettsäureestern oder Polyalkoxylierten Ethern von Fettalkoholen ausgewählt ist,
b) die Pulvermischung schmilzt, und
c) die erhaltene Schmelze zu Dosierungsformen formt.

Der Schüttwinkel (Angle of Repose) ist ein Maß für die Fließfähigkeit einer Pulvermischung. Der Schüttwinkel ist der Winkel zwischen Horizontalebene und Kegelneigung, der sich einstellt, wenn Schüttgut auf diese Ebene geschüttet wird. Eine Möglichkeit zur Bestimmung des Schüttwinkels besteht darin, die Pulvermischung in ein Gefäß zu füllen und dann eine Seitenwand zu entfernen. Ebenso ist es möglich, die Pulvermischung in einer rotierenden Trommel anzusammeln und dann den Winkel zu messen, den die Pulveroberfläche mit der Horizontalebene einschließt. Bevorzugt bestimmt man den Schüttwinkel jedoch mittels eines Schüttwinkelmessgerätes nach Dr. Pfrengle. An einem Stativ ist ein Trichter mit einem oberen Innendurchmesser von 140 mm und einem Auslauf-Innendurchmesser von 10 mm befestigt. Der Trichter umfasst eine Kurbel und einen Rührbügel, um Pulver, die nicht frei fließen, aus der Trichteröffnung zu zwängen. Mittig unter dem Trichterauslauf ist eine Basisscheibe mit einem Durchmesser von 100 mm angeordnet. Das zu prüfende Pulver wird in den Trichter eingefüllt. Bei freifließenden Pulvern wird die Ausflussöffnung freigegeben; bei zwangsfließenden Pulvern wird die Öffnung freigegeben und gleichmäßig die Kurbel des Rührbügels gedreht. Man lässt eine ausreichende Menge Pulver aus dem Trichter fließen, dass der sich bildende Schüttkegel die Basisscheibe vollständig bedeckt. Dann wird mit einem Messstab die Höhe des Pulverkegel ermittelt. Der Schüttwinkel $\alpha$ wird nach folgender Gleichung berechnet:

$$\tan \alpha = h / r$$

worin h die Höhe des Pulverkegels und r der Radius der Basisscheibe (50 mm) ist. Die Messung wird zweimal wiederholt und der Mittelwert aus den drei Einzelmessungen gebildet.

Wie die nachstehenden Beispiele und Vergleichsbeispiele zeigen, wird der Schüttwinkel der Pulvermischung durch die Mitverwendung einer Kombination aus hochdisperser Kieselsäure und wenigstens einem anorganischen Pigment drastisch verringert. Die Ursachen dafür sind nicht vollständig geklärt; vermutlich beruht der beobachtete Effekt zum einen auf der Erhöhung der Schüttdichte der Pulvermischung durch das spezifisch schwerere Pigment. Zum anderen lagern sich die Teilchen der Kieselsäure und/oder des anorganischen Pigments an der Oberfläche der größeren Bindemittelteilchen an und erhöhen deren Oberflächenrauhigkeit. Die Bindemittelteilchen können sich dadurch einander nicht mehr so stark annähern, dass sich zwischen den einzelnen Teilchen Adhäsionskräfte ausbilden können.

Vorzugsweise beträgt der Schüttwinkel einer erfindungsgemäß verwendeten Pulvermischung, die ein Bindemittel in Form eines Copolymers aus 60 Gew.-% Vinylpyrrolidon und 40 Gew.-% Vinylacetat umfasst, weniger als 39 °, vorzugsweise weniger als 38,5 °, insbesondere weniger als 37 °.

Vorzugsweise weist die Pulvermischung eine Teilchengrößenverteilung auf, worin wenigstens 50 Gew.-% der Teilchen eine Teilchengröße von weniger als 200 $\mu$m und wenigstens 10 Ges.-% der Teilchen eine Teilchengröße von weniger als 100 $\mu$m aufweisen. Insbesondere weisen wenigstens 80 Gew.-% der Teilchen eine Teilchengröße von weniger als 200 $\mu$m und wenigstens 30 Gew.-% der Teilchen eine Teilchengröße von weniger als 100 $\mu$m auf.

Die in der Pulvermischung enthaltene Menge des anorganischen Pigments ist vorzugsweise tinktorisch wirksam. Der Ausdruck "tinktorisch wirksam" soll für die Zwecke der vorliegenden Anmeldung bedeuten, dass die farbliche Erscheinung erfindungsgemäßer Dosierungsformen von der solcher Dosierungsformen, die in identischer Weise aus einer im Übrigen identischen Pulvermischung, jedoch ohne anorganisches Pigment hergestellt sind, visuell zu unterscheiden sind. Als "visuell unterscheidbar" werden z. B. Farben angesehen, denen im PANTONE Farbdefinitionssystem (Pantone Matching System@) unterschiedliche Farbzahlen zugeordnet sind.

Die Art des anorganischen Pigments ist nicht besonders kritisch, sofern es sich um ein pharmazeutisch verträgliches anorganisches Pigment handelt, das gegenüber den anderen Komponenten der Dosierungsform inert ist. Das anorganische Pigment ist vorzugsweise unter Titandioxid, insbesondere dessen Anatas-Modifikation, und Eisenoxiden, wie Goethit (gelbes Eisenoxid), Hämatit (rotes Eisenoxid) und Magnetit (schwarzes Eisenoxid), ausgewählt. Es eignen sich insbesondere solche Pigmente, die als Lebensmittelzusatz zugelassen sind, z. B. unter der Nummer E 172 der Liste der zugelassenen Lebensmittelzusätze der Europäischen Union. Die (durch Laserbeugung bestimmte) mittlere Teilchengröße des anorganischen Pigments beträgt in der Regel weniger als 50 $\mu$m, vorzugsweise weniger als 10 $\mu$m.

Die Pulvermischung enthält in der Regel 0,8 bis 5,0 Gew.-%, vorzugsweise 1 bis 4,0 Gew.-% anorganisches Pigment, bezogen auf das Gesamtgewicht der Pulvermischung.

Hochdisperse Kieselsäure ist extrem feinteiliges Siliciumdioxid, das durch Hochtemperaturhydrolyse hergestellt wird. Die Primärteilchengröße beträgt in der Regel weniger als 50 nm. Als hochdisperse Kieselsäure eignen sich insbesondere solche mit einer BET-Oberfläche von 100 bis 400 $m^2$/g, vorzugsweise 175 bis 225 $m^2$/g. Eine besonders geeignete hochdisperse Kieselsäure ist unter der Bezeichnung Aerosil® 200 erhältlich.

Unter Dosierungsformen sind alle Formen zu verstehen, die zur Verwendung als Arzneimittel, insbesondere zur oralen (besonders peroralen) Verabreichung, Pflanzenbehandlungsmittel, Futtermittel und Nahrungsergänzungsmittel geeignet sind. Dazu gehören beispielsweise Tabletten jeglicher Form, Kapseln, Pellets oder Granulate, sowie Filme und Folien.

Die Pulvermischung enthält wenigstens ein thermoplastisches Bindemittel. Dieses ist in der Regel unter Zuckeralkoholen, Derivaten davon und wasserlöslichen und wasserdispergierbaren Polymeren ausgewählt. Das Bindemittel liegt pulverförmig vor, wobei die Teilchen vorzugsweise eine gewichtsmittlere Teilchengröße im Bereich von 50 bis 1000 $\mu$m, insbesondere 100 bis 500 $\mu$m, aufweisen. Typischerweise macht das thermoplastische Bindemittel 20 bis 95 Gew.-% der Pulvermischung aus.

Geeignete thermoplastische Polymere sind beispielsweise
Polyvinylpyrrolidon (PVP),
Copolymerisate von N-Vinylpyrrolidon und Vinylacetat und/oder Vinylpropionat,
Copolymerisate von Vinylacetat und Crotonsäure,
teilverseiftes Polyvinylacetat, Polyvinylalkohol,
Polyhydroxyalkylacrylate, Polyhydroxyalkylmethacrylate,
Polyacrylate und Polymethacrylate (Eudragit®-Typen),
Copolymerisate von Methylmethacrylat und Acrylsäure,
Polyethylenglykole (Makrogol), Polyethylengylycol-Polypropylenglycol-Copolymere (Poloxamere),
Alkylcellulosen, insbesondere Methylcellulose und Ethylcellulose,
Hydroxyalkylcellulosen, insbesondere Hydroxypropylcellulose (HPC),
Hydroxyalkyl-Alkylcellulosen, insbesondere Hydroxypropylmethylcellulose (HPMC),
Celluloseester wie Cellulosephthalate, insbesondere Celluloseacetatphthalat, Hydroxypropylmethylcellulosephthalat und Hydroxypropylmethylcelluloseacetatsuccinat (HPMCAS),
Stärke, Stärkeester.

Bevorzugt verwendet man Bindemittel, die von Stärke und Stärkederivaten, wie nativer Stärke, abgebauter Stärke, vorverkleisterter Stärke oder Stärkeestern, verschieden sind.

Davon sind Homo- oder Copolymere von Vinylpyrrolidon besonders bevorzugt, z. B. Polyvinylpyrrolidon mit K-Werten nach Fikentscher von 12 bis 100, vorzugsweise 17 bis 30, oder Copolymere von 30 bis 70 Gew.-% N-Vinylpyrrolidon (VP) und 70 bis 30 Gew.-% Vinylacetat (VA), wie z. B. ein Copolymer aus 60 Gew.-% VP und 40 Gew.-% VA (Copovidon). Außerdem bevorzugt ist Hydroxypropylcellulose.

Die thermoplastischen Polymere weisen vorzugsweise eine Erweichungstemperatur von 60 bis 180 °C, insbesondere 70 bis 130 °C auf.

Geeignete Zuckeralkohole sind Sorbit, Xylit, Mannit, Maltitol; ein geeignetes Zuckeralkoholderivat ist Isomalt oder hydrierte kondensierte Palatinose wie in der DE 102 62 005 beschrieben.

Selbstverständlich können auch Gemische der genannten Bindemittel eingesetzt werden.

Die Pulvermischung enthält wenigstens einen Wirkstoff. Unter Wirkstoffen im Sinne der Erfindung sind alle Stoffe mit einer erwünschten physiologischen Wirkung auf den menschlichen oder tierischen Körper oder Pflanzen zu verstehen. Es handelt sich insbesondere um pharmazeutische Wirkstoffe. Die Wirkstoffmenge pro Dosiseinheit kann in weiten Grenzen variieren. Sie wird in der Regel so gewählt, dass sie zur Erzielung der gewünschten Wirkung ausreicht. Auch Wirkstoff-Kombinationen können eingesetzt werden. Wirkstoffe im Sinne der Erfindung sind auch Vitamine und Mineralstoffe. Zu den Vitaminen gehören die Vitamine der A-Gruppe, der B-Gruppe, worunter neben B$_1$, B$_2$, B$_6$ und B$_{12}$ sowie Nicotinsäure und Nicotinamid auch Verbindungen mit Vitamin B-Eigenschaften verstanden werden, wie z. B. Adenin, Cholin, Pantothensäure, Biotin, Adenylsäure, Folsäure, Orotsäure, Pangamsäure, Carnitin, p-Aminobenzoesäure, myo-Inosit und Liponsäure sowie Vitamin C, Vitamine der D-Gruppe, E-Gruppe, F-Gruppe, H-Gruppe, I- und J-Gruppe, K-Gruppe und P-Gruppe. Zu Wirkstoffen im Sinne der Erfindung gehören auch Peptidtherapeutika und Proteine. Zu Pflanzenbehandlungsmitteln zählen z. B. Vinclozolin, Epoxiconazol und Quinmerac.

Das erfindungsgemäße Verfahren ist beispielsweise zur Verarbeitung folgender Wirkstoffe geeignet:

Acebutolol, Acetylcystein, Acetylsalicylsäure, Aciclovir, Albrazolam, Alfacalcidol, Allantoin, Allopurinol, Ambroxol, Amikacin, Amilorid, Aminoessigsäure, Amiodaron, Amitriptylin, Amlodipin, Amoxicillin, Ampicillin, Ascorbinsäure, Aspartam, Astemizol, Atenolol, Beclomethason, Benserazid, Benzalkonium-Hydrochlorid, Benzocain, Benzoesäure, Betamethason, Bezafibrat, Biotin, Biperiden, Bisoprolol, Bromazepam, Bromhexin, Bromocriptin, Budesonid, Bufexamac, Buflomedil, Buspiron, Coffein, Campher, Captopril, Carbamazepin, Carbidopa, Carboplatin, Cefachlor, Cefalexin, Cefatroxil, Cefazolin, Cefixim, Cefotaxim, Ceftazidim, Ceftriaxon, Cefuroxim, Celedilin, Chloramphenicol, Chlorhexidin, Chlor-pheniramin, Chlortalidon, Cholin, Cyclosporin, Cilastatin, Cimetidin, Ciprofloxacin, Cisapride, Cisplatin, Clarithromycin, Clävulansäure, Clomibramin, Clonazepam, Clonidin, Clotrimazol, Codein, Cholestyramin, Cromoglycinsäure, Cyanocobalamin, Cyproteron, Desogestrel, Dexamethason, Dexpanthenol, Dextromethorphan, Dextropropoxiphen, Diazepam, Diclofenac, Digoxin, Dihydrocodein, Dihydroergotamin, Dihydroergotoxin, Diltiazem, Diphenhydramin, Dipyridamol, Dipyron, Disopyramid, Domperidon, Dopamin, Doxycyclin, Enalapril, Ephedrin, Epinephrin, Ergocalciferol, Ergotamin, Erythromycin, Estradiol, Ethinylestradiol, Etoposid, Eucalyptus Globulus, Famotidin, Felodipin, Fenofibrat, Fenofibrinsäure, Fenoterol, Fentanyl, Flavin-Mononucleotid, Fluconazol, Flunarizin, Fluorouracil, Fluoxetin, Flurbiprofen, Furosemid, Gallopamil, Gemfibrozil, Gentamicin, Gingko Biloba, Glibenclamid, Glipizid, Clozapin, Glycyrrhiza glabra, Griseofulvin, Guaifenesin, Haloperidol, Heparin, Hyaluronsäure, Hydrochlorothiazid, Hydrocodon, Hydrocortison, Hydromorphon, Ipratropium-Hydroxid, Ibuprofen, Imipenem, Indomethacin, Insulin, Iohexol, Iopamidol, Isosorbid-Dinitrat, Isosorbid-Mononitrat, Isotretinoin, Itraconazol, Ketotifen, Ketoconazol, Ketoprofen, Ketorolac, Labatalon, Lactulose, Lecithin, Levocarnitin, Levodopa, Levoglutamid, Levonorgestrel, Levothyroxin, Lidocain, Lipase, Lipramin, Lisinopril, Loperamid, Lopinavir, Lorazepam, Lovastatin, Medroxyprogesteron, Menthol, Methotrexat, Methyldopa, Methylprednisolon, Metoclopramid, Metoprolol, Miconazol, Midazolam, Minocyclin, Minoxidil, Misoprostol, Morphin, Multivitamin-Mischungen bzw. -Kombinationen und Mineralsalze, N-Methylephedrin, Naftidrofuryl, Naproxen, Neomycin, Nicardipin, Nicergolin, Nicotinamid, Nicotin, Nicotinsäure, Nifedipin, Nimodipin, Nitrazepam, Nitrendipin, Nizatidin, Norethisteron, Norfloxacin, Norgestrel, Nortriptylin, Nystatin, Ofloxacin, Omeprazol, Ondansetron, Pancreatin, Panthenol, Pantothensäure, Paracetamol, Penicillin G, Penicillin V, Phenobarbital, Phenoxifyllin, Phenoxymethylpenicillin, Phenylephrin, Phenylpropanolamin, Phenytoin, Piroxicam, Polymyxin B, Povidon-Iod, Pravastatin, Prazepam, Prazosin, Prednisolon, Prednison, Promocriptin, Propafenon, Propranolol, Proxyphyllin, Pseudoephedrin, Pyridoxin, Quinidin, Ramipril, Ranitidin, Reserpin, Retinol, Riboflavin, Rifampicin, Ritonavir, Rutosid, Saccharin, Salbutamol, Salcatonin, Salicylsäure, Simvastatin, Somatropin, Sotalol, Spironolacton, Sucralfat, Sulbactam, Sulfamethoxazol, Sulfasalazin, Sulpirid, Tamoxifen, Tegafur, Teprenon, Terazosin, Terbutalin, Terfenadin, Tetracyclin, Theophyllin, Thiamin, Ticlopidin, Timolol, Tranexamsäure, Tretinoin, Triamcinolon-Acetonid, Triamteren, Trimethoprim, Troxerutin, Uracil, Valproinsäure, Vancomycin, Verapamil, Vitamin E, Volinsäure, Zidovudin.

Das Verfahren eignet sich besonders für Wirkstoffe mit einer Löslichkeit in Wasser bei 25 °C von weniger als 1 mg/ml. Solche Wirkstoffe werden gemäß USP XXII, S. 8, auch als kaum löslich oder praktisch unlöslich bezeichnet.

Der Wirkstoff liegt in der Regel pulverförmig vor, wobei die Teilchen vorzugsweise eine gewichtsmittlere Teilchengröße im Bereich von 1 bis 1000 μm, insbesondere 5 bis 850 μm, aufweisen. Der Wirkstoff macht typischerweise 1 bis 90 Gew.-%, vorzugsweise 5 bis 60 Gew.-% der Pulvermischung aus.

Die Pulvermischung enthält wenigstens einen Solubilisator, der unter Sorbitanfettsäureestern, polyalkoxylierten Fettsäureestern oder polyalkoxylierten Ethern von Fettalkoholen ausgewählt ist.

Die Pulvermischung kann daneben verschiedene fakultative Hilfsstoffe umfassen. Pulverförmige Hilfsstoffe können einfach untergemischt werden. Flüssige oder pastöse Hilfsstoffe können zweckmäßigerweise vorab mit der Bindemittelmenge oder einem Teil davon vermengt werden, wobei man ein Granulat erhält, das in einfacher Weise mit dem Rest der Bindemittelmenge und den übrigen Komponenten der Pulvermischung vermengt werden kann. Alternativ können die Hilfsstoffe in die Schmelze eingearbeitet werden, z. B. über Dosierstellen im Zylinder des Extruders.

Derartige fakultative Hilfsstoffe sind:

Solubilisatoren, wie Sorbitanfettsäureester, polyalkoxylierte Fettsäureester, wie z. B polyalkoxylierte Glyceride, polyalkoxylierte Sorbitanfettsäureester oder Fettsäureester von Polyalkylenglykolen; oder polyalkoxylierte Ether von Fettalkoholen. Eine Fettsäurekette in diesen Verbindungen umfasst in der Regel 8 bis 22 Kohlenstoffatome. Die Polyalkylenoxid-Blöcke umfassen pro Molekül durchschnittlich 4 bis 50 Alkylenoxideinheiten, vorzugsweise Ethylenoxideinheiten.

Geeignete Sorbitanfettsäureester sind Sorbitanmonolaurat, Sorbitanmonopalmitat, Sorbitanmonostearat, Sorbitanmonooleat, Sorbitantristearat, Sorbitantrioleat, Sorbitanmonostearat, Sorbitanmonolaurat oder Sorbitanmonooleat.

Geeignete polyalkoxylierte Sorbitanfettsäurester sind beispielsweise Polyoxyethylen(20)sorbitanmonolaurat, Polyoxyethylen(20)sorbitanmonopalmitat, Polyoxyethylen(20)sorbitanmonostearat, Polyoxyethylen(20)sorbitanmonooleat, Polyoxyethy-len(20)sorbitantristearat, Polyoxyethylen(20)sorbitantrioleat, Polyoxyethylen(4)sorbitanmonostearat, Polyoxyethylen(4)sorbitanmonolaurat oder Polyoxyethylen(4)sorbitanmonooleat.

Geeignete polyalkoxylierte Glyceride werden z. B. durch Alkoxylierung von natürlichen oder hydrierten Glyceriden bzw. durch Umesterung von natürlichen oder hydrierten Glyceriden mit Polyalkylenglykolen erhalten. Handelsübliche Beispiele sind Polyoxyethylenglycerolricinoleat-35, Polyoxyethylenglyceroltrihydroxystearat-40 (Cremophor® RH40, BASF AG) sowie polyalkoxylierte Glyceride wie sie unter den Handelsnamen Gelucire® und Labrafil® von Gattefosse erhältlich sind, z. B. Gelucire® 44/14 (Lauroyl-Macrogol-32-glyceride, hergestellt durch Umesterung von hydriertem Palmkernöl mit PEG 1500), Gelucire® 50/13 (Stearoyl-Macrogol-32-glyceride, hergestellt durch Umesterung von hydriertem Palmöl mit PEG 1500) oder Labrafil M1944 CS (Oleoyl-Macrogol-6-glyceride, hergestellt durch Umesterung von Aprikosenkernöl mit PEG 300).

Ein geeigneter Fettsäureester von Polyalkylenglykolen ist z. B. PEG-660-Hydroxystearinsäure (Polyglykolester der 12-Hydroxystearinsäure (70 mol-%) mit 30 mol-% Ethylenglykol).

Geeignete polyalkoxylierte Ether von Fettalkoholen sind z. B. Macrogol-6-Cetylstearylether oder Macrogol-25-Cetylstearylether

Solubilisatoren werden typischerweise in einer Menge von 0,1 bis 15 Gew.-%, vorzugsweise 0,5 bis 10 Gew.-% in der Pulvermischung mitverwendet. Solubilisatoren mit wenigstens einer Polyalkoxyeinheit im Molekül sind bevorzugt.

Sprengmittel, wie vernetztes Polyvinylpyrrolidon und vernetzte Natriumcarboxymethylcellulose.

Streckmittel bzw. Füllstoffe, wie Lactose, Cellulose, Silikate oder Kieselsäure,

Schmiermittel, wie Magnesium- und Calciumstearat, Natriumstearylfumarat,

Farbstoffe, wie Azofarbstoffe, organische oder anorganische Pigmente oder Farbstoffe natürlicher Herkunft,

Stabilisatoren, wie Antioxidanzien, Lichtstabilisatoren, Hydroperoxid-Vernichter, Radikalfänger, Stabilisatoren gegen mikrobiellen Befall.

Zur Herstellung der festen Dosierungsformen wird bei einer erhöhten Temperatur, d. h. einer Temperatur bei oder oberhalb des Erweichungspunkts des Bindemittels eine Schmelze, d. h. eine formbare kohäsive Masse aus der Pulvermischung hergestellt, die anschließend, gegebenenfalls nach einem Formgebungsschnitt, abgekühlt wird. Vorzugsweise beträgt die Zeitspanne, über die die Komponenten der erhöhten Temperatur ausgesetzt sind, für jede der Komponenten weniger als 5 Minuten, insbesondere weniger als 3 Minuten.

Das Vermischen der Komponenten zur Pulvermischung erfolgt in üblichen Mischern, wie Pflugscharmischern, Schüttel- bzw. Freifallmischern und dergleichen. Zur Herstellung der Pulvermischung kann man auch eine Vormischung aus einzelnen Komponenten und/oder Teilmengen der Komponenten herstellen und die restlichen Komponenten und/oder die Restmengen einzelner Komponenten zu einem späteren Zeitpunkt untermischen. So kann man beispielsweise eine wirkstofffreie Vormischung auf Vorrat herstellen und einen oder mehrere Wirkstoffe bei Bedarf untermischen. Alternativ kann es vorteilhaft sein, eine Vormischung herzustellen, die die Wirk- und Hilfsstoffe und gegebenenfalls eine Teilmenge des Bindemittels enthält, und diese Vormischung in das Bindemittel und/oder die Hauptmenge des Bindemittels einzuarbeiten.

Das Erwärmen erfolgt in einer für diesen Zweck üblichen Vorrichtung. Besonders geeignet sind beheizbare Extruder oder Kneter, wie Misch-Knetreaktoren (z. B. ORP, CRP, AP, DTB der Firma List oder Reactotherm der Firma Krauss-Maffei oder Ko-Kneter der Fa. Buss), Doppelmuldenkneter (Trogmischer) und Stempelkneter (Innenmischer) oder Rotor/Stator-Systeme (z. B. Dispax der Firma IKA).

Als Extruder kann man Einschneckenmaschinen, kämmende Schneckenmaschinen oder auch Mehrwellextruder, insbesondere Zweischnecken-Extruder, gleichsinnig oder gegensinnig drehend und gegebenenfalls mit Knetscheiben ausgerüstet, einsetzen. Besonders bevorzugt sind Doppelschneckenextruder der ZSK-Baureihe von Werner u. Pfleiderer.

Das Beschicken des Extruders bzw. Kneters erfolgt je nach deren Konzeption kontinuierlich oder diskontinuierlich in üblicher Weise. Die Pulvermischung wird vorzugsweise im freien Zulauf, z. B. über eine Differentialdosierwaage eingeführt werden.

Die erhaltene Schmelze ist teigig bis pastös. Sie wird in der Regel einer Formgebung unterzogen. Dabei kann eine Vielzahl von Formen, je nach Werkzeug und Art der Formung, erzeugt werden. Beispielsweise lässt sich bei Verwendung eines Extruders der extrudierte Strang zwischen einem Band und einer Walze, zwischen zwei Bändern oder zwischen zwei Walzen, wie in der EP-A-358 105 beschrieben, oder durch Kalandrierung in einem Kalander mit zwei Formwalzen, siehe beispielsweise EP-A-240 904, formen. Durch Extrusion und Heiß- oder Kaltabschlag des Stranges können beispielsweise kleinteilige Granulate erhalten werden.

Die erkalteten Massen können anschließend auch zu Pulver gemahlen und dann in üblicher Weise zu Tabletten verpresst werden. Hierbei können Tablettierhilfsmittel wie kolloidale Kieselsäure, Calciumhydrogenphosphat, Lactose, mikrokristalline Cellulose, Stärke oder Magnesiumstearat mitverwendet werden.

Die Erfindung wird durch die folgenden Beispiele näher veranschaulicht.

Beispiele 1 bis 3

10,5 Gew.-Teile Copovidon wurden in einem Labor-Mischgranulator (Fa. Bohle) innerhalb von 5 min mit 2 Gew.-Teilen Solubilisator (Labrafil M 1944 CS) vermischt. Das erhaltene Granulat wurde innerhalb von 40 min mit 71,5 Gew.-Teilen Copovidon, 15,0 Gew.-Teilen Fenofibrat und 1,0 Gew.-Teil hochdisperser Kieselsäure (Aerosil 200) gemischt. Aliquote der Pulvermischung wurden in einem Schüttelmischer (TURBULA) innerhalb von 2,5 min homogen mit den in Tabelle 1 angegebenen Mengen Titandioxid (Anatas; mind. 98 % < 10 μm) gemischt; die Eigenschaften der erhaltenen Mischungen sind in Tabelle 1, die durch Siebanalyse ermittelte Teilchengrößenverteilung in Tabelle 2 zusammengefasst (angegeben ist der prozentuale Anteil, der durch ein Sieb mit der angegebenen Maschenweite verbleibt). Die Schüttdichte bestimmte man mittels eines Stampfvolumeters JEL der Firma Engelsmann, Ludwigshafen, Deutschland.

Tabelle 1:

| Beispiel | Titandioxid [Gew.-%] | Schüttdichte [g/ml] | Schüttwinkel [°] |
|---|---|---|---|
| 1* | 0 | 0,27 | 40,14 |
| 2 | 1 | 0,29 | 38,30 |
| 3 | 5 | 0,34 | 35,34 |
| *Vergleichsbeispiel | | | |

Tabelle 2:

| Beispiel | Pfanne | 100 μm | 200 μm | 315 μm | 500 μm | 630 μm | 1000 μm |
|---|---|---|---|---|---|---|---|
| 1* | 0,3 | 9,3 | 85,6 | 3,7 | 0,5 | 0,3 | 0,5 |
| 2 | 36,4 | 52,1 | 9,0 | 2,0 | 0,1 | 0,2 | 0 |
| 3 | 53,9 | 35,4 | 8,7 | 2,3 | 0,4 | 0,3 | 0 |
| *Vergleichsbeispiel | | | | | | | |

Die Pulvermischungen wurden in den Fülltrichter eines Doppelschneckenextruder (ZSK 25; Werner & Pfleiderer) eingefüllt und bei einer Extrusionstemperatur von 80-155 °C extrudiert. Der Durchsatz betrug 5 kg/h beim Beispiel 1 und 7-8 kg/h beim Beispiel 3. Man schnitt das Extrudat in Stücke und ließ es erstarren. Die Extrudatstücke wurde in einer schnelllaufenden Mühle (Comil) vermahlen. Das Mahlgut (97,64 Gew.-Teile) wurde in einem TURBULA-Mischer innerhalb von 5 min mit Natriumstearylfumarat (1,30 Gew.-Teile) und hochdisperser Kieselsäure (Aerosil 200; 1,06 Gew.-Teile) vermengt. Aus dem Gemisch stellte man auf einer Exzenter-Tablettenpresse (Fette E 1) Tabletten von 54 mg bzw. 160 mg Wirkstoffgehalt her.

Die Auflösungsgeschwindigkeiten der erhaltenen Tabletten in einer 0,05 M Lösung von Natriumdodecylsulfat (75 U/min) sind in den Tabellen 3A und 3B zusammengestellt.

Tabelle 3A: Wirkstoffauflösung [%] Tablette 54 mg

| Beispiel | 10 min | 20 min | 30 min | 40 min | 50 min | 60 min |
|---|---|---|---|---|---|---|
| 1* | 26,5 | 45,4 | 60,8 | 76,6 | 87,1 | 92,6 |
| 2 | 25,8 | 45,5 | 61,4 | 78,7 | 87,6 | 98,3 |

(fortgesetzt)

| Beispiel | 10 min | 20 min | 30 min | 40 min | 50 min | 60 min |
|---|---|---|---|---|---|---|
| 3 | 26,1 | 49,1 | 67,8 | 84,5 | 96,8 | 100 |
| *Vergleichsbeispiel | | | | | | |

Tabelle 3B: Wirkstoffauflösung [%] Tablette 160 mg

| Beispiel | 10 min | 20 min | 30 min | 40 min | 50 min | 60 min |
|---|---|---|---|---|---|---|
| 1* | 10,5 | 25,8 | 36,3 | 50,4 | 62,1 | 75,6 |
| 2 | 18,6 | 34,0 | 46,7 | 59,3 | 69,1 | 85,3 |
| 3 | 26,9 | 44,9 | 56,6 | 69,7 | 79,2 | 88,0 |
| *Vergleichsbeispiel | | | | | | |

Man sieht, dass die Auflösungsgeschwindigkeit mit steigendem Gehalt an Titandioxid zunimmt, wobei der Effekt bei höherem Tablettengewicht stärker ausgeprägt ist.

Beispiele 4 bis 6

Die Beispiele 1 bis 3 wurden wiederholt, wobei man an Stelle des Copovidon Hydroxpropylmethylcellulose (HPMC) verwendete. Die Eigenschaften der erhaltenen Pulvermischungen sind in Tabelle 4 zusammengefasst.

Tabelle 4:

| Beispiel | Titandioxid [Gew.-%] | Schüttdichte [g/ml] | Schüttwinkel [°] |
|---|---|---|---|
| 4* | 0 | 0,35 | 46,49 |
| 5 | 1 | 0,36 | 43,43 |
| 6 | 5 | 0,38 | 41,99 |
| *Vergleichsbeispiel | | | |

Beispiel 7

150,0 g Fenofibrat, 715,0 g des in Tabelle 5 genannten Matrixpolymers, 125,0 g Labrafil M 1944 CS und 10 g Aerosil 200 wurden in einem Schüttelmischer (TURBULA) innerhalb von 2 min bei 30 UpM homogen gemischt. Die so erhaltene Vormischung wurde über ein 2 mm-Sieb (Frewitt S1B) abgesiebt. Die Endmischung wurde in einem Schüttelmischer (TURBULA) 3 min bei 30 UpM gemischt. Außerdem stellte man entsprechende Mischungen her, die 10,0 g bzw. 50,0 g Titandioxid enthielten; die Menge Matrixpolymer wurde um diesen Betrag verringert. Die Eigenschaften der erhaltenen Pulvermischungen sind in Tabelle 5 zusammengefasst.

Tabelle 5: Schüttwinkel von Pulvermischungen

| Titandioxid Gew.-% | HPC | Maisstärke | Poloxamer | Macrogol 6000 | K 30 | K30/ HPMC 1:1 | K30/ HPMC 2:1 |
|---|---|---|---|---|---|---|---|
| 0 | 44,0* | 47,8* | 32,3* | 44,7* | 32,5* | 36,7* | 35,4* |
| 1 | 42,2 | 44,7 | 30,0 | 44,2 | 32,2 | 37,0 | 34,5 |
| 5 | 40,4 | 43,0 | 33,2 | 42,5 | 29,1 | 35,0 | 32,9 |
| *Vergleichsbeispiel<br>HPC: Hydroxypropylcellulose<br>K30: Polyvinylpyrrolidon (K-Wert nach Fikentscher 30)<br>HPMC: Hydroxypropylmethylcellulose | | | | | | | |

**Patentansprüche**

1. Feste Dosierungsform, umfassend eine Matrix mit wenigstens einem homogen in der Matrix dispergierten Wirkstoff, die durch Schmelzen einer Pulvermischung erhältlich ist, wobei die Pulvermischung wenigstens ein thermoplastisches Bindemittel, eine Kombination aus hochdisperser Kieselsäure und wenigstens einem anorganischen Pigment und außerdem wenigstens einen Solubilisator enthält, der unter Sorbitanfettsäureestern, polyalkoxylierten Fettsäureestern oder polyalkoxylierten Ethern von Fettalkoholen ausgewählt ist.

2. Dosierungsform nach Anspruch 1, wobei der Wirkstoff in der Matrix in Form einer esten Lösung vorliegt.

3. Dosierungsform nach Anspruch 1 oder 2, wobei die Pulvermischung eine tinktorisch wirksame Menge des anorganischen Pigments enthält.

4. Dosierungsform nach einem der vorhergehenden Ansprüche, wobei das anorganische Pigment unter Titandioxid und Eisenoxiden ausgewählt ist.

5. Dosierungsform nach Anspruch 4, wobei das Titandioxid in der Anatas-Modifikation vorliegt.

6. Dosierungsform nach einem der vorhergehenden Ansprüche, wobei das anorganische Pigment eine mittlere Teilchengröße von weniger als 50 $\mu$m aufweist.

7. Dosierungsform nach einem der vorhergehenden Ansprüche, wobei die Pulvermischung 0,8 bis 5,0 Gew.-% anorganisches Pigment enthält.

8. Dosierungsform nach einem der vorhergehenden Ansprüche, wobei die Pulvermischung 0,5 bis 2,0 Gew.-% hochdisperse Kieselsäure enthält.

9. Dosierungsform nach einem der vorhergehenden Ansprüche, wobei das thermoplastische Bindemittel unter Zuckeralkoholen, Derivaten davon und wasserlöslichen und wasserdispergierbaren Polymeren ausgewählt ist.

10. Dosierungsform nach Anspruch 9, wobei das thermoplastische Bindemittel unter Homo- oder Copolymeren von Vinylpyrrolidon, Polyalkylenoxiden, Stärke, Hydroxypropylcellulose, Hydroxpropylmethylcellulose und Gemischen davon ausgewählt ist.

11. Verfahren zur Herstellung fester Dosierungsformen, bei dem man

a) eine Pulvermischung bereitstellt, die wenigstens ein thermoplastisches Bindemittel, eine Kombination aus hochdisperser Kieselsäure und wenigstens einem anorganischen Pigment und außerdem wenigstens einen Solubilisator enthält, der unter Sorbitanfettsäureestern, polyalkoxylierten Fettsäureestern oder polyalkoxylierten Ethern von Fettalkoholen ausgewählt ist,
b) die Pulvermischung schmilzt, und
c) die erhaltene Schmelze zu Dosierungsformen formt.

12. Verfahren nach Anspruch 11, bei dem man die erkaltete Schmelze zu Teilchen vermahlt und die Teilchen zu Dosierungsformen verpresst.

**Claims**

1. Solid dosage form comprising a matrix with at least one active compound dispersed homogeneously in the matrix, which is obtainable by melting a powder mixture, wherein the powder mixture comprises at least one thermoplastic binder, a combination of highly disperse silica and at least one inorganic pigment and furthermore at least one solubilizer, which is chosen from sorbitan fatty acid esters, polyalkoxylated fatty acid esters or polyalkoxylated ethers of fatty alcohols.

2. Dosage form according to Claim 1, wherein the active compound is present in the matrix in the form of a solid solution.

3. Dosage form according to Claim 1 or 2, wherein the powder mixture comprises a tinctorially active amount of the inorganic pigment.

4. Dosage form according to one of the preceding claims, wherein the inorganic pigment is chosen from titanium dioxide and iron oxides.

5. Dosage form according to Claim 4, wherein the titanium dioxide is present in the anatase modification.

6. Dosage form according to one of the preceding claims, wherein the inorganic pigment has an average particle size of less than 50 $\mu$m.

7. Dosage form according to one of the preceding claims, wherein the powder mixture comprises 0.8 to 5.0 wt.% of inorganic pigment.

8. Dosage form according to one of the preceding claims, wherein the powder mixture comprises 0.5 to 2.0 wt.% of highly disperse silica.

9. Dosage form according to one of the preceding claims, wherein the thermoplastic binder is chosen from sugar alcohols, derivatives thereof and water-soluble and water-dispersible polymers.

10. Dosage form according to claim 9, wherein the thermoplastic binder is chosen from homo- or copolymers of vinylpyrrolidone, polyalkylene oxides, starch, hydroxypropylcellulose, hydroxypropylmethylcellulose and mixtures thereof.

11. Process for the preparation of solid dosage forms, in which

   a) a powder mixture which comprises at least one thermoplastic binder, a combination of highly disperse silica and at least one inorganic pigment and furthermore at least one solubilizer, which is chosen from sorbitan fatty acid esters, polyalkoxylated fatty acid esters or polyalkoxylated ethers of fatty alcohols, is provided,
   b) the powder mixture is melted, and
   c) the melt obtained is shaped into dosage forms.

12. Process according to Claim 11, in which the cooled melt is ground to particles and the particles are pressed to dosage forms.

**Revendications**

1. Forme galénique solide comprenant une matrice avec au moins un principe actif dispersé de manière homogène dans la matrice, qui peut être obtenue par fusion d'un mélange pulvérulent, où le mélange pulvérulent contient au moins un liant thermoplastique, une combinaison d'acide silicique hautement dispersé et d'au moins un pigment inorganique et en outre au moins un solubilisant qui est choisi parmi les esters d'acides gras et de sorbitane, les esters d'acides gras polyalcoxylés ou les éthers d'alcools gras polyalcoxylés.

2. Forme galénique selon la revendication 1, où le principe actif est présent dans la matrice sous forme d'une première solution.

3. Forme galénique selon la revendication 1 ou 2, où le mélange pulvérulent contient une quantité efficace du point de vue tinctorial du pigment inorganique.

4. Forme galénique selon l'une des revendications précédentes où le pigment inorganique est choisi parmi le dioxyde de titane et les oxydes de fer.

5. Forme galénique selon la revendication 4, où le dioxyde de titane est présent dans la modification anatase.

6. Forme galénique selon l'une des revendications précédentes, où le pigment inorganique présente une taille de particule moyenne inférieure à 50 $\mu$m.

7. Forme galénique selon l'une des revendications précédentes, où le mélange pulvérulent contient 0,8 à 5,0 % en poids de pigment inorganique.

**8.** Forme galénique selon l'une des revendications précédentes, où le mélange pulvérulent contient 0,5 à 2,0 % en poids d'acide silicique hautement dispersé.

**9.** Forme galénique selon l'une des revendications précédentes, où le liant thermoplastique est choisi parmi les sucres-alcools, leurs dérivés et les polymères solubles dans l'eau et dispersibles dans l'eau.

**10.** Forme galénique selon la revendication 9, où le liant thermoplastique est choisi parmi les homo- ou copolymères de vinylpyrrolidone, les poly(oxydes d'alkylène), l'amidon, l'hydroxypropylcellulose, l'hydroxypropylméthylcellulose et leurs mélanges.

**11.** Procédé pour produire des formes galéniques solides, dans lequel on

a) prépare un mélange pulvérulent qui contient au moins un liant thermoplastique, une combinaison d'acide silicique hautement dispersé et d'au moins un pigment inorganique et en outre au moins un solubilisant qui est choisi parmi les esters d'acides gras et de sorbitane, les esters d'acides gras polyalcoxylé ou les éthers d'alcools gras polyalcoxylés,
b) fait fondre le mélange pulvérulent, et
c) met la masse fondue obtenue sous forme de formes galéniques.

**12.** Procédé selon la revendication 11, dans lequel on broie la masse fondue refroidie en particules et compresse les particules en formes galéniques.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 240904 A
- EP 240906 A
- EP 337256 A
- EP 358105 A
- WO 9820863 A
- WO 0134121 A
- DE 3325130 A1
- WO 9215285 A
- US 5427614 A
- DE 10262005

**In der Beschreibung aufgeführte Nicht-Patentliteratur**

- **ZIMMERMANN, I. et al.** *Powder Technology,* 2004, vol. 139, 40-54